# EUROPEAN PATENT APPLICATION

(11) **EP 2 272 891 A2**
(43) Date of publication of application: **12.01.2011**
(21) Application number: 10251220.9
(22) Date of filing: 07.07.2010
(51) Int. Cl.: C08G 65/332, A61B 17/072, A61B 17/115, A61B 17/15

(54) **Surgical gasket**

(30) Priority: 08.07.2009 US 499146
(71) Applicant: Tyco Healthcare Group, LP, New Haven CT 06511 (US)
(72) Inventor: Calabrese, Allison, Memphis, TN 38103 (US); Hadba, Ahmad Robert, Middlefield, CT 06455 (US); Skalla, Walter, Old Lyme, CT 06371 (US)
(74) Representative: Soames, Candida Jane

(57) **Abstract**

A gasket for use with a surgical stapling apparatus is disclosed. The gasket comprises a diisocyanate end-capped poly(ether ester). The diisocyanate is selected from the following: aromatic, aliphatic and acyclic isocyanates. The poly(ether ester) can further comprise at least one polyol and at least one poly acid. The gasket is desirably bioabsorbable.

## Description

### CROSS-REFERENCE TO RELATED APPLICATIONS

The present application is a continuation-in-part of U.S. Patent Application Serial No. 12/351,492 filed January 9, 2009, by Reinprecht et al., which is a continuation-in part of U.S. Patent Application no. 11/123,690, filed May 5, 2005, by Hadba et al., the disclosures of each of which are hereby incorporated by reference herein in their entirety.

### BACKGROUND

### Technical Field

The present disclosure relates to gaskets for use in conjunction with surgical fastening devices, for reducing occurrences of leaking, bleeding and/or stricture.

### Background of Related Art

Staples have traditionally been used instead of suturing when joining or anastomosing various body structures, such as, for example, the bowel or bronchus. The surgical stapling devices employed to apply these staples are generally designed to simultaneously cut and seal an extended segment of tissue in a patient, thus vastly reducing the time and risks of such procedures.

Linear or annular surgical stapling devices are employed by surgeons to sequentially or simultaneously apply one or more linear rows of surgical fasteners, e.g., staples or two-part fasteners, to body tissue for the purpose of joining segments of body tissue together and/or for the creation of anastomoses. Linear surgical stapling devices generally include a pair of jaws or finger-like structures between which body tissue to be joined is placed. When the surgical stapling device is actuated and/or "fired," a firing mechanism moves longitudinally and actuates staple drive members in one of the jaws, and surgical staples are pushed through the body tissue and into/against an anvil in the opposite jaw thereby crimping the staples closed. A knife blade may be provided to cut between the rows/lines of staples.

Annular or circular surgical stapling devices, such as an end-to-end anastomosis stapler, generally include an annular staple cartridge assembly including a plurality of annular rows of staples, typically two, an anvil assembly operatively associated with the annular cartridge assembly, and an annular blade disposed internal of the rows of staples. Examples of such annular surgical stapling devices are described in U.S. Patent Nos. 5,915,616 to Viola et al. and 7,168,604 to Milliman et al., the entirety of each of which is incorporated herein by reference.

For most procedures, the use of staples alone, with the staples in direct contact with the patient's tissue, is generally acceptable. The integrity of the tissue will normally serve to prevent the staples from tearing out of the tissue and compromising the sealing before healing has occurred. However, in some surgical operations, surgical supports, e.g., reinforcing materials, gaskets, meshes or buttresses, are employed by surgeons to bridge, repair and/or reinforce tissue defects with a patient, especially those occurring in the abdominal wall, chest wall, diaphragm and other musculo-aponeurotic areas of the body.

When the staples are applied in surgical procedures utilizing surgical supports, the legs of the staple typically pass from the cartridge jaw through a layer of the surgical support, and through the patient's tissue before encountering the anvil jaw. In an alternative procedure, the legs of the staple typically pass from the cartridge jaw through a first layer of the surgical support, then through the patient's tissue, and finally through a second layer of the surgical support before encountering the anvil jaw. With the staples in place, the stapled tissue is clamped between the layers of the surgical support.

In addition to the use of surgical staples, biological tissue adhesives have been developed for tissue repair and the creation of anastomoses. Generally, biological adhesives bond separated tissues together to aid in the healing process and to enhance the tissue strength. Such adhesives may be used instead of suturing and stapling, for example, in surgical procedures, for the repair of tissue or the creation of anastomoses.

### SUMMARY

The present disclosure relates to a gasket for use with a surgical stapling apparatus, the gasket comprising a diisocyanate end-capped poly(ether ester). The diisocyanate may be selected from the group consisting of aromatic, aliphatic and acyclic isocyanates.

In certain embodiments, the diisocyanate is selected from the group consisting of 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), tetramethylxylylene diisocyanate, tetramethylene diisocyanate, hexamethylene diisocyanate, dimethyl diisocyanate, lysine diisocyanate, 2-methylpentane-1,5-diisocyanate, 3-methylpentane-1,5-diisocyanate or 2,2,4-trimethylhexamethylene diisocyanate, isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate and combinations thereof.

The poly(ether ester) may comprise at least one polyol and at least one polyacid. The at least one polyol is selected from the group consisting of polyalkylene oxides, polyvinyl alcohols, polysaccharides, and combinations thereof, in certain embodiments.

The at least one polyacid may be an aliphatic polyacid. In certain embodiments, the aliphatic polyacid is selected from the group consisting of sebacic acid, azelaic acid, suberic acid, pimelic acid, adipic acid, glutaric acid, succinic acid, malonic acid, oxalic acid and combinations thereof.

In certain preferred embodiments, the gasket is bioabsorbable. The gasket may be absorbed in the body of from about 1 day to about 21 days. The gasket may be tear-resistant, elastic, compliant, and /or swellable. The may be formed from a curable material. The gasket may be formed from a UV curable material.

In certain preferred embodiments, at least a portion of the gasket is porous.

The gasket may further comprise a wound treatment material.

In certain embodiments, the gasket comprises a pre-cured adhesive or sealant.

The gasket may comprise a material selected from the group consisting of diisocyanate end-capped poly(ether esters), polyesters, polyamides, polyurethanes, polypeptides, proteins, nucleic acids, polysaccharides and combinations thereof.

In a further aspect, a gasket for use with a surgical stapling apparatus is disclosed. The gasket comprising: a body portion having an upper surface and a lower surface, the body portion defining a central longitudinal axis extending between the upper surface and the lower surface, body portion comprising a diisocyanate end-capped poly(ether ester); a plurality of pores extending through the body portion, wherein each of the pores extends from the upper surface to the lower surface such that each pore defines an axis that is substantially parallel to the central longitudinal axis; the body portion having an outer region disposed outwardly of the plurality of pores, wherein the outer region is substantially non-porous.

In certain embodiments, the diisocyanate is lysine diisocyanate, hexamethylene diisocyanate, or phenylene diisocyanate.

In certain preferred embodiments, the body portion is dimensioned so that the plurality of pores is positioned at a staple line of the surgical stapling apparatus and the outer region is positioned outwardly of the staples fired by the surgical stapling apparatus.

The the body portion may have an outer terminal edge, and the body portion may be dimensioned so that the outer terminal edge is positioned outwardly of an outer perimeter of an anvil assembly of the surgical stapling apparatus.

In certain embodiments, the plurality of pores is formed by cutting the body portion, or formed by stamping.

The body portion may include an outer terminal edge, and an inner terminal edge defining an aperture therein. The aperture may be dimensioned and arranged to receive a shaft of a surgical stapling apparatus.

In certain preferred embodiments, the body portion is substantially circular in shape.

The body portion may include polyethylene glycol. The body portion may be elastic. The body portion may be swellable.

### BRIEF DESCRIPTION OF DRAWINGS

Various embodiments of the presently disclosed surgical gasket are disclosed herein with reference to the drawings, wherein:

FIG. 1 is a perspective view of an annular surgical stapling device in accordance with an embodiment of the present disclosure;

FIG. 1A is a perspective view of a linear surgical stapling device in accordance with an embodiment of the present disclosure;

FIGS. 2A and 2B are plan, and transverse cross-sectional views, respectively, of an annular gasket in accordance with an embodiment of the present disclosure;

FIGS. 3A and 3B are plan, and transverse cross-sectional views, respectively, of an annular gasket in accordance with an embodiment of the present disclosure;

FIGS. 4A and 4B are plan, and transverse cross-sectional views, respectively, of an annular gasket in accordance with an embodiment of the present disclosure;

FIGS. 5A and 5B and plan, and transverse cross-sectional views, respectively, of a linear gasket in accordance with an embodiment of the present disclosure;

FIG. 6 is a transverse cross-sectional view of an annular gasket in accordance with an embodiment of the present disclosure;

FIG. 7 is a plan view of an annular gasket in accordance with an embodiment of the present disclosure;

FIG. 8 is a perspective view of the intestinal area of a patient, illustrating a method of using the annular surgical stapling device of FIG. 1;

FIG. 9 is a longitudinal cross-sectional view of a portion of the surgical stapling device of FIG. 1 in a non-approximated position, illustrating the annular gasket of FIGS. 2A and 2B positioned on an anvil rod and two layers of tissue adjacent the anvil rod; and

FIG. 10 is a longitudinal cross-sectional view of a portion of the surgical stapling device of FIG. 1 in an approximated position, illustrating the annular gasket of FIGS. 2A and 2B positioned on the anvil rod and two layers of tissue adjacent the anvil rod.

### DETAILED DESCRIPTION OF EMBODIMENTS

Embodiments of the presently disclosed annular gaskets will now be described in detail with reference to the drawings wherein like reference numerals identify similar or identical elements. As used herein and as is traditional, the term "distal" refers to that portion which is farther from the user while the term "proximal" refers to that portion which is closer to the user. As used herein, the term "gasket" includes staple line reinforcement material, buttress, pledget, or any other sheet or pad of material used in conjunction with surgical fastening apparatus.

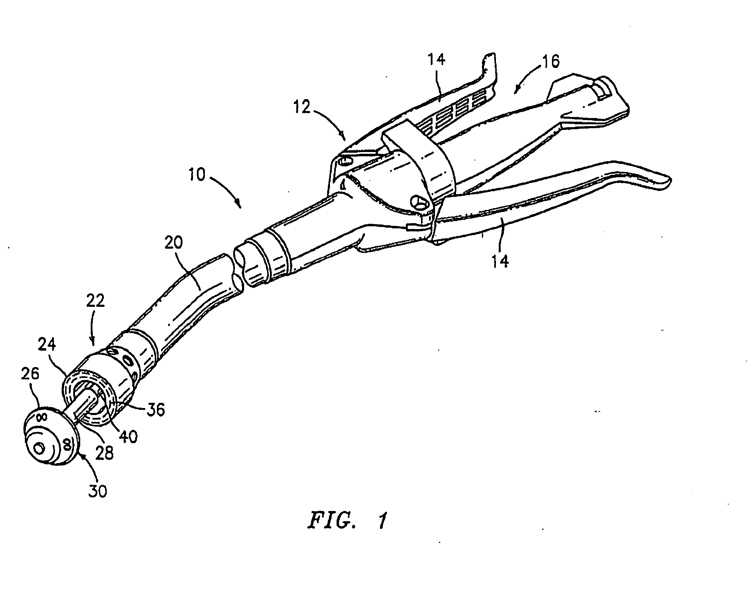

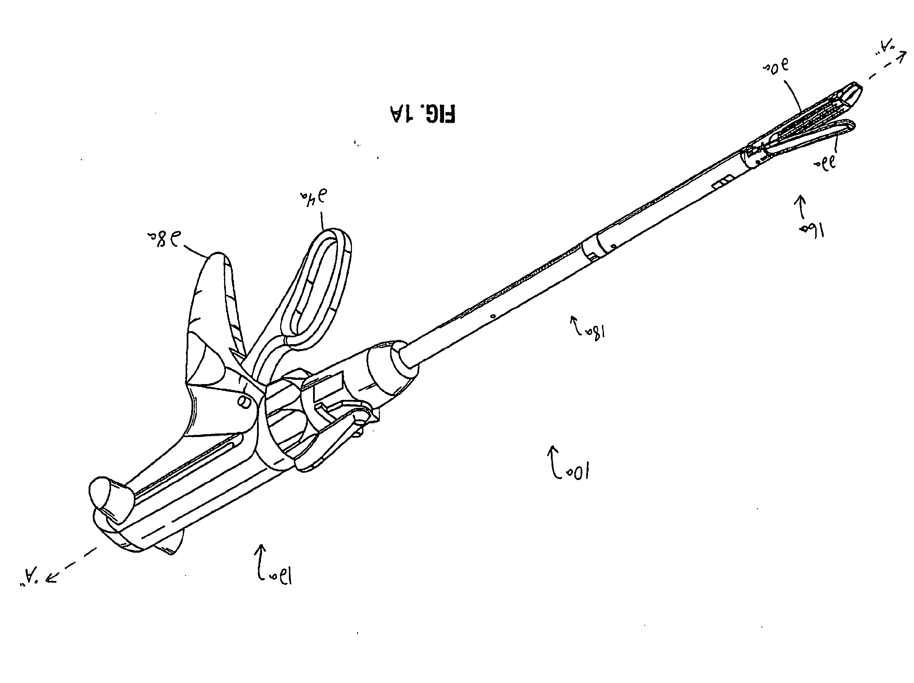

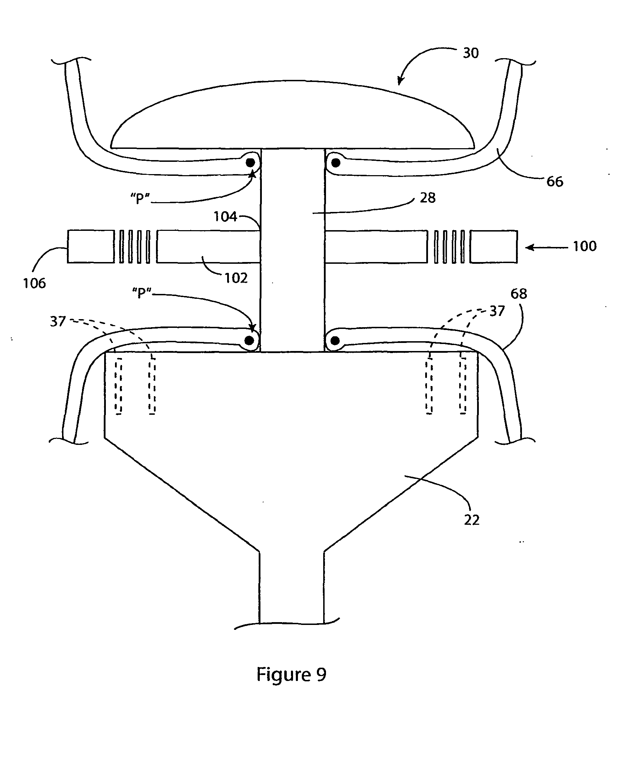

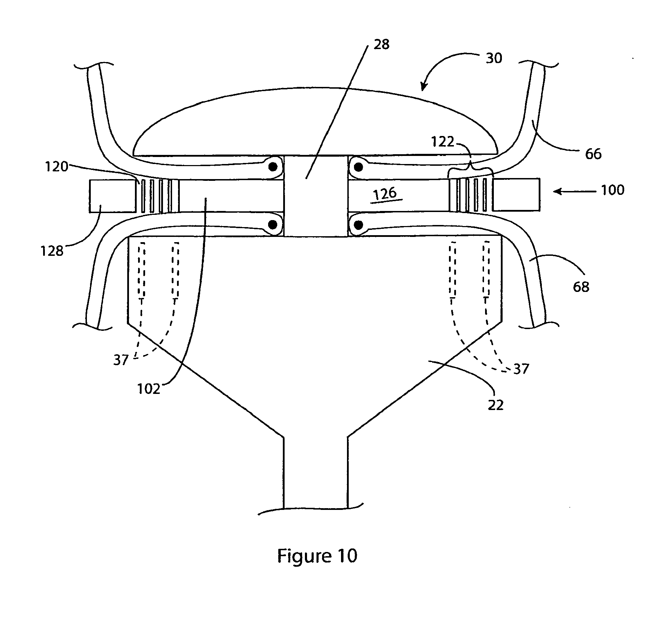

Referring initially to FIG. 1, an annular surgical stapling device, for use with the annular gaskets disclosed herein, is generally designated as 10. Surgical stapling device 10 includes a handle assembly 12 having at least one pivotable actuating handle member 14, and an advancing member 16. Extending from handle member 12, there is provided a tubular body portion 20 which may be constructed so as to have a curved shape along its length. Body portion 20 terminates in a staple cartridge assembly 22 which includes a pair of annular arrays of staple receiving slots 36 having a staple 37 (schematically shown in FIGS. 9 and 10) disposed in each one of staple receiving slots 36. Positioned distally of staple cartridge assembly 22 there is provided an anvil assembly 30 including an anvil member 26 and a shaft 28 operatively associated therewith for removably connecting anvil assembly 30 to a distal end 40 of tubular body portion 20.

Staple cartridge assembly 22 may be fixedly connected to the distal end 40 of tubular body portion 20 or may be configured to concentrically fit within the distal end 40 of tubular body portion 20. Typically, staple cartridge assembly 22 includes a staple pusher (not shown) including a proximal portion having a generally frusto-conical shape and a distal portion defining two concentric rings of peripherally spaced fingers (not shown), each one of which is received within a respective staple receiving slot 36.

Typically, a knife (not shown), substantially in the form of an open cup with the rim thereof defining a knife edge, is disposed within staple cartridge assembly 22 and mounted to a distal surface of a staple pusher (not shown). The knife edge is disposed radially inward of the pair of annular arrays of staples. Accordingly, in use, as the staple pusher is advanced, the knife is also advanced axially outward.

Reference may be made to U.S. Patent Nos. 5,915,616 to Viola et al. and 7,168,604, the entire contents of each of which have been incorporated by reference hereinabove, for a detailed discussion of various annular stapling devices.

Referring to FIG. 1A, a linear surgical stapling device for use with the linear gaskets disclosed herein is generally designated as 10a. Linear surgical stapling device 10a includes a handle assembly 12a near a proximal end, an end effector 16a near a distal end and an elongate portion 18a therebetween. The end effector 16a may be positioned within a body cavity to engage tissue at a surgical site while handle assembly 12a is manipulatable by a surgeon from outside the body cavity to control the movement and operation of the end effector 16a. Elongate portion 18a defines a longitudinal axis "A-A."

End effector 16a includes a cartridge assembly 20a, which houses a plurality of staples (not shown) arranged in linear rows, and an anvil assembly 22a for forming the staples. At least one of the cartridge assembly 20a and the anvil assembly 22a is movable with respect to the other between an open position wherein the cartridge assembly 20a is substantially spaced from the anvil assembly 22a and an approximated position where the cartridge assembly 20a and the anvil assembly 22a are closer together. A pivotable trigger 24a of the handle assembly 12a is movable through an actuation stroke or strokes relative to a stationary grip member 28a to move cartridge assembly 20a in relation to anvil assembly 22a between the open position and the approximated position, to eject the staples from cartridge assembly 20a, and/or to advance a knife to cut tissue. Further details of a linear surgical stapling device are described in detail in commonly-owned U.S. Patent No. 6,953,139 to Milliman et al., the entire contents of which are hereby incorporated by reference herein.

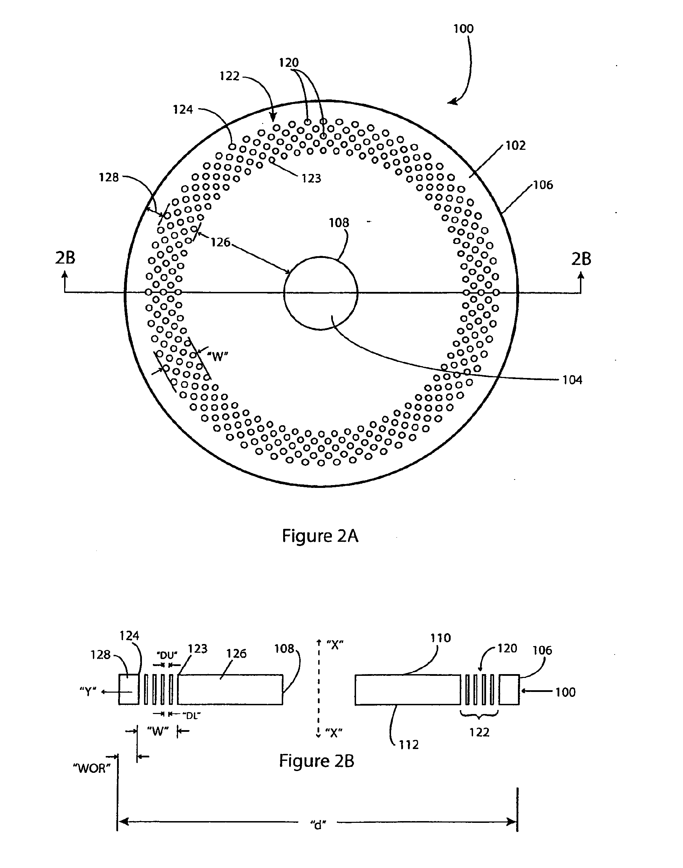

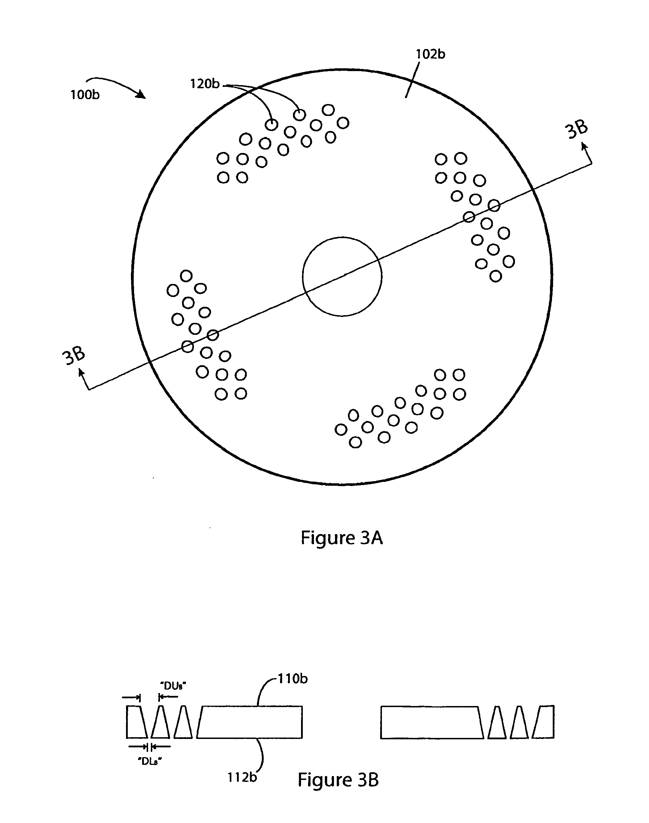

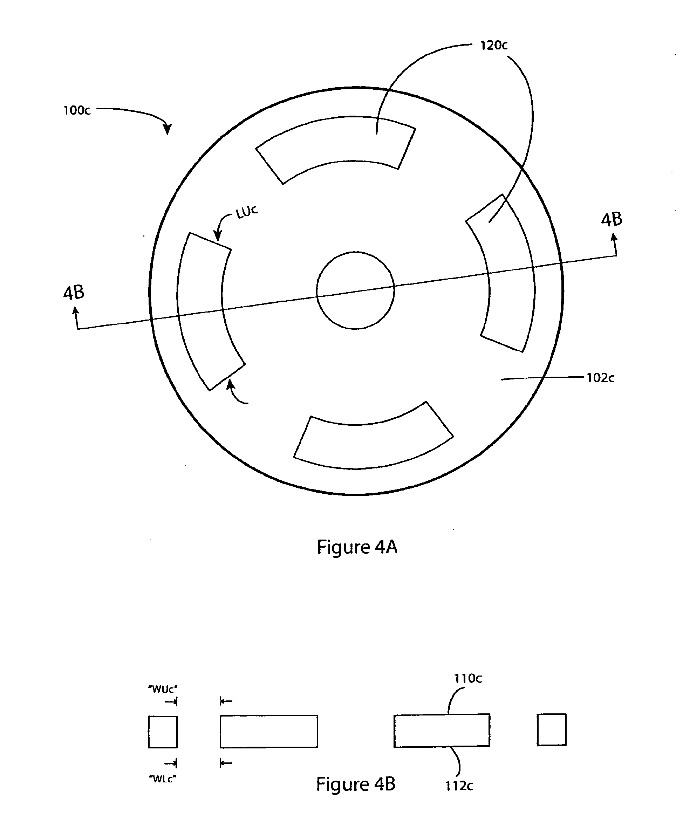

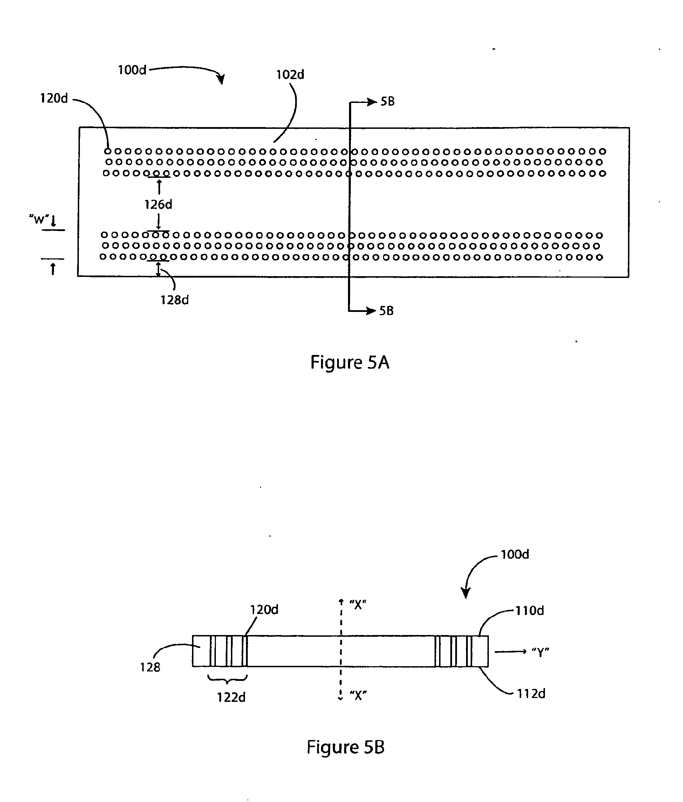

Turning now to FIGS. 2 - 4, an annular gasket, in accordance with various embodiments of the present disclosure is illustrated. For clarity, the embodiment of the gasket illustrated in FIGS. 2A and 2B, gasket is designated as numeral 100; the gasket illustrated in FIGS. 3A and 3B is designated as numeral 100b; and the gasket illustrated in FIGS. 4A and 4B is designated as numeral 100c. Additionally, similar features of gaskets 100, 100b and 100c are discussed with reference to gasket 100 of FIGS. 2A and 2B. The gasket 100d of FIGS. 5A and 5B is discussed separately below. As can be appreciated, various features of gaskets 100, 100b and 100c may be common with gasket 100d, and the description of such features with respect to gasket 100d may be omitted herein for clarity.

Gasket 100 includes a washer- or disk-like body portion 102 including a substantially centrally located aperture 104 formed therethrough. Gasket 100 is defined by an outer terminal edge 106, an inner terminal edge 108 defining the size of aperture 104, an upper surface 110, and a lower surface 112. Body portion 102 defines a central longitudinal axis "X-X" (see FIG. 2B, for example) extending through aperture 104. As discussed in further detail below, the use of gasket 100 may help reduce the likelihood of leaking, bleeding and/or stricture adjacent an anastomosis joint.

In various embodiments, gasket 100 is sized such that when gasket 100 is operatively associated with stapling device 10, as will be described in greater detail below, outer terminal edge 106 extends radially outwardly of staple retaining pockets 36 of staple cartridge assembly 22. Additionally, aperture 104 of gasket 100 is sized to at least receive shaft 28 of anvil assembly 30 therethrough. It is also envisioned that the distance between outer terminal edge 106 and inner terminal edge 108 is substantially equal to a width of a tissue contact surface 24 (see FIG. 1) of staple cartridge assembly 22.

The gasket 100 includes a plurality of pores 120 disposed through the body portion 102, such that each pore defines an axis that is substantially parallel to axis "X-X." The pores in certain embodiments are uni-directional. Pores 120 allow for ingrowth of intestinal sections 66, 68 therethrough. In particular, pores 120 of body portion 102 reduce the time required for intestinal sections 66, 68 to contact one another during the healing process. For example, depending on the size of pores 120, intestinal sections 66, 68 may contact one another immediately following the firing of surgical stapling device 10, or pores 120 define channels into which intestinal sections 66, 68 may grow and come into contact with one another over time.

The pores 120 of the gasket 100 define a porous area 122. It is envisioned that the location of the porous area 122, with respect the aperture 104, is approximately aligned with the location of the staples 37 (shown schematically in FIGS. 9 and 10) that are configured to penetrate the gasket 100. As can be appreciated, in such embodiments, each staple penetrates a portion of the porous area 122. It is further envisioned that the width "W" of the porous area 122 is approximately equal to the width of the staple line, which may be about 3 mm.

As discussed below with reference to the embodiments in FIGS. 2-4, the porous area 122 of the gasket 100 may include a relatively large amount of relatively small pores 120 (e.g., FIGS. 2A and 2B), a relatively medium amount of relatively medium sized pores 120b (e.g., FIGS. 3A-3B), a relatively small amount of relatively large pores 120c (e.g., FIGS. 4A-4B), or any other suitable configuration. In each embodiment, the inclusion of pores 120 in body portion 102 may be accomplished by various methods, such as, for example, laser cutting, salt leaching, hot pressing or stamping. The pores 120 may be defined by a woven material or mesh, or may be discrete openings formed in a sheet of material. Accordingly, it is envisioned that body portion 102 (i.e., inclusive of porous area 122), an inner region 126 (discussed below) and an outer region 128 (discussed below) is made from a single material. The pores 120 may be punched or laser-cut in any shape or pattern. The die may be shaped according to the desired shape (or shapes) of the pores 120, or the laser may be rastered to form shapes. Alternatively, the porous area 122 and outer region 128 can be formed from separate materials that are joined, connected or fused together.

The gasket 100 also includes an inner region 126 and an outer region 128. Inner region 126 is the area of gasket 100 disposed between the inner terminal edge 108 and an inner edge 123 of the porous area 122. Outer region 128 is the area of gasket 100 disposed between an outer edge 124 of the porous area 122 and outer terminal edge 106. At least a portion of inner region 126 is within the staple line and thus will be dissected with the tissue. That is, after firing of knife, at least a portion of inner region 126 will be cut away from the remainder of body portion 102 of gasket 100 and will pass through the patient's body. The inner region 126, in certain embodiments, comprises a solid inner portion that will allow for placement on a surgical fastening device, such a circular stapler.

Outer region 128 is solid (i.e., substantially non-porous) and will remain in place after firing of staples and knife. More specifically, due to its non-porosity, outer region 128 prevents leakage of fluids (e.g., body fluids) in the radial outward direction along arrow "Y" (see FIG. 2B, for example), which is substantially perpendicular to axis "X-X." Additionally, outer region 128 prevents leakage of fluids in radial outward directions that are angularly disposed with respect to arrow "Y."

It is envisioned that the width "W_{OR}" of outer region 128 relates to the diameter of the stapler cartridge. It is envisioned that the diameter "d" of gasket 100 is greater than the diameter defined by the outer row of staples 37 in the cartridge assembly 22 and anvil assembly 30, such that at least a portion of outer region 128 extends radially beyond the outer staple row and in some embodiments outward of cartridge assembly 22 and anvil assembly 30. The outer region 128, and the size of the gasket 100 generally, should be arranged so that the outer row of staples catches some material in the outer region 128 to ensure the integrity of the gasket 100 in an anastomosis.

With reference to FIGS. 2-4, various types of gaskets 100 are contemplated by the present disclosure. The difference between each gasket 100 in FIGS. 2-4 relates its pores 120 and porous area 122. More particularly, the placement, size and/or shape of the pores 120 vary with respect to the different embodiments.

With particular reference to FIGS. 2A and 2B, gasket 100 includes plurality of pores 120 that substantially continuously extend around the upper surface 110 (and correspondingly extend around the lower surface 112) of the body portion 102. While FIG. 2A illustrates the pores 120 extending substantially continuously around the body portion 102 (i.e., one section of pores), it is envisioned that the pores 120 are disposed in at least two sections that are spaced from one another. For example, pores 120 may be disposed in four sections, such as the four sections of pores 120b and 120c illustrated in FIGS. 3A and 4A, respectively.

It is envisioned that each pore 120 includes a diameter at the upper surface 110 that is larger than the diameter at the lower surface 112, and it is also envisioned that each pore has a diameter at the upper surface that is smaller than the diameter at the lower surface. Furthermore, some pores can have their larger diameters at the upper surface and some pores can have their larger diameters at the lower surface. It is also envisioned that the diameters of the pores 120 differ from one another (i.e., that not all pores 120 have the same diameter). It is also envisioned that the pores have a trapezoidal, rectangular or other regular or irregular shape in the plan and/or cross-sectional views of Figs. 2A through 7 discussed above.

With particular reference to FIG. 2B, the transverse cross-sectional view (taken along arrow 2B-2B of FIG. 2A) of gasket 100 depicts, *inter alia,* the size of select pores 120. More particularly, FIG. 2B illustrates the diameter of various pores 120 at the upper surface 110 ("DU") and at the lower surface 112a ("DL"). In FIG. 2B, these two diameters are shown as being substantially equal, but it is envisioned that one of these diameters is larger than the other (see the embodiment illustrated in FIG. 3B, for example).

With reference to FIGS. 3A and 3B, the gasket illustrated in FIGS. 3A and 3B is generally designated as reference numeral 100b. Gasket 100b includes plurality of pores 120b that extend around the upper surface 110b (and correspondingly extend around the lower surface 112b) of the body portion 102b in four sections. While FIG. 3A illustrates the pores 120b extending around the body portion 102b in four sections, it is envisioned that the pores 120b are disposed in more or fewer sections. For example, pores 120b may be disposed in one section, such as the one sections of pores 120 illustrated in FIG. 2A.

With particular reference to FIG. 3B, the transverse cross-sectional view (taken along arrow 3B-3B of FIG. 3A) of gasket 100b depicts, *inter alia,* the size of select pores 120b. More particularly, FIG. 3B illustrates the diameter of various pores 120b at the upper surface 110b ("DU_{B}") and at the lower surface 112b ("DL_{B}"). In FIG. 3B, diameter DU_{B} is shown as being larger than diameter DL_{B}. As can be appreciated, such a configuration of pores 120b may facilitate tissue growth from the portion of tissue adjacent upper surface 110b towards the portion of tissue adjacent lower surface 112b. Accordingly, tissue growth from the portion of tissue adjacent lower surface 112b towards the portion of tissue adjacent upper surface 110b is limited or substantially prevented. While, FIG. 3B illustrates unequal-diameter pores 120b, it is envisioned that diameters DU_{B} and DL_{B} are substantially equal, as shown in the embodiments illustrated in FIGS. 2B and 4B, for example. It is also envisioned that diameter DU_{B} is smaller than diameter DL_{B}. It is further envisioned that a single gasket 100 includes some pores 120 having a larger diameter DL_{U} than diameter DL_{B}, and some pores 120 having a larger diameter DL_{B} than diameter DL_{U}.

With reference to FIGS. 4A and 4B, the gasket illustrated in FIGS. 4A and 4B is generally designated as reference numeral 100c. Gasket 100c includes plurality of pores 120c that extend around the upper surface 110c (and correspondingly extend around the lower surface 112c) of the body portion 102c in four sections. That is, in this embodiment, each pore 120c makes up a single section. While FIG. 4A illustrates the pores 120c extending around the body portion 102c in four sections, it is envisioned that the pores 120c are disposed in more or fewer sections. For example, pores 120c may be disposed in three or five sections.

With particular reference to FIG. 4B, the transverse cross-sectional view (taken along arrow 4B-4B of FIG. 4A) of gasket 100c depicts, *inter alia,* the size of select pores 120c. More particularly, FIG. 4B illustrates the width of various pores 120c at the upper surface 110c ("WU_{C}") and at the lower surface 112c ("WL_{C}"). In FIG. 4B, these two widths are shown as being substantially equal, but it is envisioned that one of these widths is larger than the other (see the embodiment illustrated in FIG. 3B, for example).

Referring now to FIGS. 5A and 5B, linear gasket 100d is illustrated. Similar to gaskets 100, 100b and 100c, gasket 100d includes a body portion 102d, an upper surface 110d, a lower surface 112d, a plurality of pores 120d, and a porous area 122d. Additionally, gasket 100d includes an inner region 126d disposed between porous areas 122d, and a solid outer region 128d, which is disposed outwardly (i.e., transversely outwardly) of each porous area 122d.

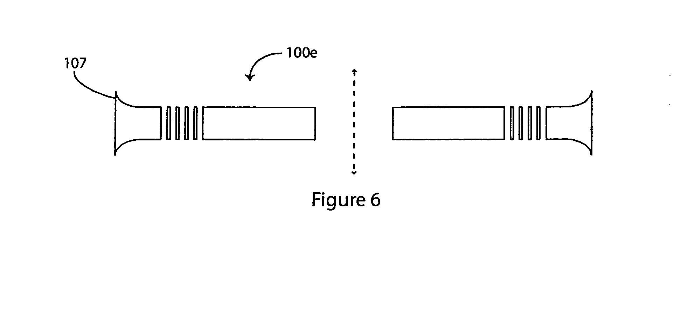

With reference to FIG. 6, in a further embodiment, gasket 100e is similar in construction as gasket 100a (shown in FIGS. 2A and 2B), except that an outer edge 107 of the gasket 100e is flared. Thus, the gasket 100e is as described above in connection with FIGS. 2A and 2B except that the outer edge 107 is flared as shown in FIG. 6. The flared shape contributes to forming a seal around the area where the two tissue/intestinal sections are joined in an anastomosis.

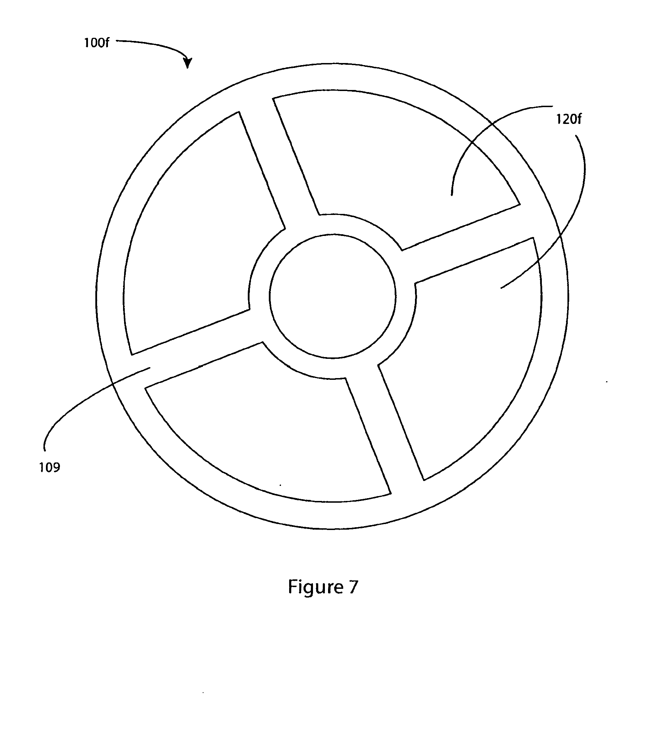

In a further embodiment, and with reference to FIG. 7, gasket 100f is similar in construction as gasket 100c (shown in FIGS. 4A and 4B, except that the pores 120f are larger, defining spokes of material 109 therebetween. Thus, the gasket is as described above in connection with FIGS. 4A and 4B except that the pores 120f are larger to encourage tissue ingrowth. The pores 120f are elongate in a circumferential direction and generally trapezoidal in shape. The circumferential direction is along the circumference of a circular or elliptical gasket. Pores of other shapes are contemplated, including triangular, rectangular, elliptical, or any polygon.

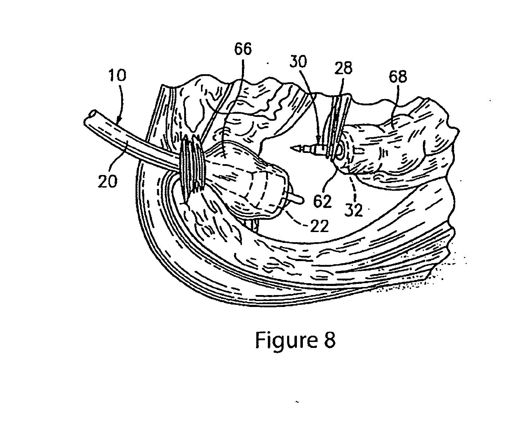

Turning now to FIGS. 8-10, the use of surgical stapling device 10 and gasket 100 (FIGS. 9 and 10) in an anastomosis procedure to join intestinal sections 66 and 68 of tissue is illustrated. The anastomosis procedure is typically performed using minimally invasive surgical techniques including laparoscopic means and instrumentation. At the point in the procedure shown in FIG. 8, a diseased intestinal section has been previously removed, anvil assembly 30 has been introduced to the operative site either through a surgical incision or transanally and positioned within intestinal section 68. Additionally, tubular body portion 20 of surgical stapling device 10 has been inserted transanally into intestinal section 66. Intestinal sections 66 and 68 are also shown temporarily secured about their respective components (e.g., the distal end of tubular body portion 20, and shaft 28 of anvil assembly 30, respectively) by conventional means such as a purse string suture "P" (see FIGS. 9 and 10).

Referring generally to FIGS. 9 and 10, gasket 100 is placed onto shaft 28 of anvil assembly 30 prior to the coupling of anvil assembly 30 to the distal end of tubular body portion 20. In particular, it is envisioned that shaft 28 of anvil assembly 30 is inserted into aperture 104 of body portion 102. Following positioning of gasket 100 onto shaft 28 of anvil assembly 30, and with particular reference to FIG. 9, the surgeon maneuvers anvil assembly 30 until the proximal end of shaft 28 is inserted into the distal end of tubular body portion 20 of surgical stapling device 10, wherein the mounting structure (not shown) within the distal end of tubular body portion 20 engages shaft 28 to effect the mounting. It is also envisioned that gasket 100 includes a radial slit (not shown) therein extending from aperture 104 through outer terminal edge 106. Such a radial slit would enable gasket 100 to be placed onto shaft 28 of anvil assembly 30 after the mounting structure engages shaft 28.

Thereafter, as shown in FIG. 10, following approximation of anvil assembly 30 and tubular body portion 20 to approximate intestinal sections 66, 68 and capture body portion 102 of gasket 100 therebetween, surgical stapling device 10 may be fired thereby stapling intestinal sections 66, 68 to one another and cutting the portion of tissue and gasket 100 disposed radially inward of the knife (i.e., inner region 126), to complete the anastomosis. Following the anastomosis, tissue ingrowth occurs through pores 120 (e.g., at the staple line), while the solid outer region 128 prevents occurrences of radial leakage at the site of the anastomosis.

It is envisioned that at least one portion of gasket 100 (e.g., porous area 122) is coated with a fast-absorbing biocompatible material (such as, for example collagen, gelatin, chitosan, carboxymethyl cellulose, fast absorbing polymers or copolymers of lactide, glycolide, caprolactone, poly(alkylene oxides) such poly(ethylene oxide) or its copolymers with poly(propylene oxide), polyester-ether urethanes and/or ureas and combinations thereof) to increase its cohesive strength. It is envisioned that such a coating is applied by solvent coating, spray coating, electrospinning, electrospraying, etc., for example.

In disclosed embodiments, gasket 100 is made from a material (or a combination of materials) that is sufficiently strong so as to not be damaged by the force of the stapling device 10, and that is at least as compliant as the stapled tissue. Additionally, it is envisioned that gasket 100 is made from a material (or a combination of materials) that will swell after contacting bodily fluids, such that the pressure created by the swelling will help seal the anastomosis and possible enhance the leak prevention functions of gasket 100. Accordingly, suitable materials that can be used to make gasket 100 and/or its coating include diisocyanate end-capped poly(ether esters), polyesters, polyamides, polyurethanes, polypeptides, proteins, nucleic acids, and polysaccharides. Suitable poly(ether esters) include polymers or oligomers prepared from at least one polyol and at least one polyacid. Suitable polyols include but are not limited to polyalkylene oxides, polyvinyl alcohols, polysaccharides, and the like. In some embodiments, the polyhydroxy compounds can be a polyalkylene oxide such as polyethylene oxide ("PEO"), polypropylene oxide ("PPO"), block or random copolymers of polyethylene oxide (PEO) and polypropylene oxide (PPO). Suitable aliphatic polyacids include, but are not limited to sebacic acid, azelaic acid, suberic acid, pimelic acid, adipic acid, glutaric acid, succinic acid, malonic acid, oxalic acid and combinations thereof. Suitable polyesters can be such as based on copolymers of polyalkylene oxides and glycolide, glycolic acid, lactide, lactic acid, caprolactone, p-dioxanone, trimethylene carbonate, and combinations thereof.

The diisocyanates of interest include aromatic, aliphatic and alicyclic isocyanates. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate) or tetramethylxylylene diisocyanate; aliphatic diisocyanates such as tetramethylene diisocyanate, hexamethylene diisocyanate, dimethyl diisocyanate, lysine diisocyanate, 2-methylpentane-1,5-diisocyanate, 3-methylpentane-1,5-diisocyanate or 2,2,4-trimethylhexamethylene diisocyanate; and alicyclic diisocyanates such as isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate or commercially available DESMODURS^{®} from Bayer Material Science.

It is also envisioned that the polyurethanes are chain extended using low molecular weight diols, diamines or alcoholamines. Suitable chain extenders may include but are not limited to ethylene glycol, propylene glycol, butane diol, hexane diol, octane diol, poly(ethylene glycol), hydroxyl-terminated polyalkylene oxides such as PEG and copolymers of ethylene glycol and propylene glycol, ethylene diamine, hexamethylene diamine, lysine, spermine, spermidine, N-(3-aminopropyl)-1,4-butanediamine, N,N'-bis(3-aminopropyl)-1,4-butanediamine, isomers of hexamethylene diamine, diethylene triamine, triethylene tetramine, tetraethylene pentamine, bishexamethylene triamine, N,N'-bis(3-aminopropyl)-1,2-ethane diamine, N-(3-Aminopropyl)-1,3-propane diamine, N-(2-aminoethyl)-1,3 propane diamine, cyclohexane diamine, isomers of cyclohexane diamine, 4,4'-methylene biscyclohexane amine, 4'4'-methylene bis(2-methylcyclohexanamine), toluene diamine, phenylene diamine, isophorone diamine, phenalkylene polyamines, amino-functionalized polyalkylene oxides such as PEG and copolymers of ethylene glycol and propylene glycol, polypeptides and combinations thereof. Additionally, positively charged beads may be incorporated into the polymer to accelerate the wound healing cascade.

It is also envisioned that gasket 100 may be impregnated with a wound treatment material, e.g., an adhesive, sealant, and/or other medicament. Accordingly, in use, the sealant component of gasket 100 may function to retard bleeding which may occur from the tissue. The wound treatment material of gasket 100 may function to secure the approximated tissue together. In embodiments where gasket 100 is bio-absorbable, the bio-absorbability of gasket 100 would allow for the at least a portion of gasket 100 to be absorbed into the body after a predetermined amount of time. For example, gasket 100 may remain in place in the body for approximately 2-3 weeks in order for the anastomosis to sufficiently heal prior to gasket 100 being absorbed into the body.

Other materials that may be used for body gasket 100 include, and are not limited to, those fabricated from homopolymers, copolymers or blends obtained from one or more monomers selected from the group consisting of glycolide, glycolic acid, lactide, lactic acid, p-dioxanone, α-caprolactone and trimethylene carbonate. Other bio-absorbable materials include and are not limited to, for example, Polyglycolic Acid (PGA) and Polylactic Acid (PLA). In disclosed embodiments, gasket 100 may be fabricated from bio-absorbable felt, gelatin or any other bio-absorbable materials.

It is contemplated that the adhesive may be a biocompatible adhesive, including, and not limited to, adhesives which cure upon tissue contact, which cure upon exposure to ultraviolet (UV) light, which are two-part systems which are kept isolated from one another and cure upon coming into contact with one another, which are pressure sensitive, which are any combinations thereof, or any other known suitable adhesive. In one embodiment, it is contemplated that an adhesive having a cure time of from about 10 seconds to about 15 seconds may be used. In another embodiment, it is contemplated that an adhesive having a cure time of about 30 seconds may be used.

It is envisioned that gasket 100 may be impregnated with a pre-cured adhesive or sealant. The pre-cured adhesive or sealant will react with the moisture and/or heat of the body tissue to thereby activate the sealing and/or adhesive properties of the sealant or adhesive. It is envisioned that the pre-cured sealant or adhesive may be a hydro-gel or the like.

Other surgically biocompatible wound treatment materials which may be employed in or applied by surgical instruments, especially surgical staplers, include adhesives whose function is to attach or hold organs, tissues or structures; sealants to prevent fluid leakage; hemostats to halt or prevent bleeding; and medicaments. Examples of adhesives which can be employed include protein derived, aldehyde-based adhesive materials, for example, the commercially available albumin/glutaraldehyde materials sold under the trade designation BioGlue^{™} by Cryolife, Inc., and cyanoacrylate-based materials sold under the trade designations Indermil^{™} and Derma Bond^{™} by Covidien and Ethicon Endosurgery, Inc., respectively. Examples of sealants, which can be employed, include fibrin sealants and collagen-based and synthetic polymer-based tissue sealants. Examples of commercially available sealants are synthetic polyethylene glycol-based, hydrogel materials sold under the trade designation CoSeal^{™} by Cohesion Technologies and Baxter International, Inc. Examples of hemostat materials, which can be employed, include fibrin-based, collagen-based, oxidized regenerated cellulose-based and gelatin-based topical hemostats. Examples of commercially available hemostat materials are fibrinogen-thrombin combination materials sold under the trade designations CoStasis^{™} by Tyco Healthcare Group, LP, Crosseal™ fibrin sealant sold by Johnson & Johnson, and Tisseel^{™} sold by Baxter International, Inc. Hemostats herein include astringents, e.g., aluminum sulfate, and coagulants.

The medicament may be disposed on gasket 100 or impregnated therein. The medicament may include one or more medically and/or surgically useful substances such as drugs, enzymes, growth factors, peptides, proteins, dyes, diagnostic agents or hemostasis agents or any other pharmaceutical used in the prevention of stenosis.

In disclosed embodiments, it is contemplated that gasket 100 may be impregnated with a first component of a two-part adhesive and that the staples, retained in staple receiving slots 36 of staple cartridge assembly 22, may be coated with a second component (e.g., a reactant) of the two-part adhesive. In this manner, the first component of the adhesive is activated when the staples penetrate and capture body portion 102 of support 100 during the firing sequence of surgical stapling device 10, and the two components of the adhesive contact one another.

The present disclosure also relates to various methods of manufacturing a gasket 100. Disclosed methods include the steps of providing a non-porous body portion 102 having upper surface 110, and lower surface 112; and creating a plurality of pores 120 through the body portion 102. Each of the pores 120 extend from upper surface 110 to lower surface 112 such that each pore 120 defines an axis that is substantially parallel to the central longitudinal axis. Each of the plurality of pores 120 is created by at least one of the group of consisting of laser cutting, salt leaching, hot pressing and stamping.

The present disclosure also relates to a surgical stapling kit. The kit includes a surgical stapling apparatus 10, 10a, as described above, and gasket 100, as described above.

The gasket may include a hub for attaching the gasket to a circular stapler. The gasket is preferably attached to a position on the anvil shaft or tubular body portion shaft so that the gasket is positioned between the sections of tissue to be joined. A hub for attaching a gasket to a circular stapling instrument is disclosed in U.S. Published Patent Application No. 2007/0203509 A1, the disclosure of which is hereby incorporated by reference herein. Alternatively, the gasket can be incorporated with the shaft and deployed therefrom. A deployable gasket is disclosed in U.S. Published Patent Application No. 2006/0135992 AI, the disclosure of which is hereby incorporated by reference herein.

In further embodiments, the gasket is semi-circular in shape. Such a gasket can be used with a surgical stapler like that shown in FIG. 1, except that the anvil and body portion are semi-circular in shape, or a surgical stapler that deploys staples in a sequential manner from curved jaws. The disclosure of U.S. Patent Application No. 12/235,751 is hereby incorporated herein and discloses a surgical stapler with hook-shaped jaws. A rectangular-shaped gasket may be used with a linear surgical stapler, such as the surgical staplers disclosed in U.S. Patent Nos. 5,318,221 and 5,782,396 and 6,953,139, the disclosures of which are all hereby incorporated by reference herein.

In a further aspect, a polymeric material for a gasket according to any of the embodiments discussed above is disclosed. The material is desirably tear-resistant, swellable in use, and very elastic and compliant. Preferably, the material is elastic and compliant in the radial direction of the gasket. It is desirable if the material is fairly rigid when dry. Poly ether urethane urea materials are disclosed. For example, (PEG-Ad-PEG-HMDI-BD) may be used, where PEG is polyethylene glycol, Ad is adipate, HMDI is hexamethylene diisocyanate, and BD is butane diol. The HMDI may be replaced with Lysine or PDI, where PDI is phenylene diisocyanate. Examples of such materials are disclosed in U.S. Patent Publication No. 2006/0253094, entitled Bioabsorbable Surgical Composition, the entire contents of which are hereby incorporated by reference herein.

An example of a polyurethane is made in one pot without having to purify in between steps. Materials are: 1601 g/mol PEG adipate (lot 433s-49), MDI (purified by dissolution in toluene, filtration, evaporation) (Aldrich lot 06823EE), hexanediol 20% with v in DMF, and DMF. Weigh at 3.4924 grams MDI in a clean, dry 100 ml RB flask equipped with a mechanical stirrer. Place under blanket N₂. Heat to 80 degrees Celsius and set stirring at 50 rpm. Weigh out P2 (the 433s-49material?yes) for a 1.98 to 1 molar ratio of MDI to PEG adipate. Add 11.282 grams of the P2 slowly over the course of about 1 minute. Set Stirring to 100 rpm and let react 2 hours at temperature. Add .825 grams HD. When mixture is thick (white and foamy) add 25 ml DMF to dissolve. Let react ½ hour total. Pour into Teflon dish to dry in a hood. The material is stretchy. Once swollen in water, the material is relatively strong.

Another one pot example uses the following materials: 1601 g/mol PEG adipate (lot 433 s -49), MDI (purified by dissolution in toluene, filtration and evaporation) (Aldrich lot 06823EE), butanediol 20% in DMF, and DMF. Following the same procedure as above, the material can also be swollen in water.

In a further example, the materials used are: 34.97 grams PEG (lot 4335-16) (1767 g/mol), .988 grams (1,4) butenediol, 5.04 grams purified PDI (solvent purified), triethylamine (as catalyst). Mix the diob and dry under bubbling N₂ at 40 degrees Celsius overnight at 25 rpm. The following day, add 1 drop of TEA, raise the temperature to 65 degrees Celsius, and add PDI. Mix at temperature for 15 minutes, pour into Teflon tray and place in 100 degrees Celsius overnight to cure.

In another example, the following were used: PEG adipate functionalized with PDI (427 s -136), butanediol (20% solution in DMF with v), stannous octoate, and DMF. Add 12.89 grams of the 4275-136 material and 1 drop stennous to a clean dry 2N 100 ml RB flask with a stir shaft and Teflon blade. Heat the mixture up to 80 degrees Celsius, mixing at 50 rpm. Add butandiol (want equimolar NCO:OH). Add 3 ml of DMF solution, stir at 185 rpm and heat. The material will solidify upon stirring. Add 50 ml DMF and heat to 90 degrees Celsius to dissolve. Pour into Teflon tray to dry. The material will be relatively elastic and strong.

In a further example, add the following materials to a clean, dry 100 ml RB flask with a mechanical stirrer: 6.9226 grams of PCL diol 1250, .5037 grams butanediol, 2.5142 grams distilled LDI (lysine diisocyanate), and I drop Sn(Oct)_{2.} Stir at 50 rpm and attach static N₂ to flask. Put in oil bath and set bath to 80 degrees Celsius. After 1- ½ hours, the material is white in color and thick. Add 15 ml DMF and dissolve quickly. Allow the reaction to proceed at 80 degrees Celsius overnight. Dry the solution under a vacuum. The dried solution is rigid and waxy and does not seem to swell.

Add the following materials to a 100 ml 2 neck RB flask: 19.753 grams P2 (lot 433s-63, 1608 grams/ mol) dried 1 hour at 65 degrees Celsius and 4 days under bubbling N₂, 8.575 grams LDI distilled 5/08, and 1 drop Sn(Oct)₂. Attach a mechanical stirrer and blanket N₂. Place in oil bath. Stir at 100 rpm and set oil bath at 65 degrees Celsius. Let react for 21 hours. Attach a water- cooled spiral condenser and perform 9 extractions with petroleum ether, stirring at 400-500 rpm. For each extraction, add about 30 ml petroleum ether, mix for 2-3 minutes, then take off the ether with a pipet. Final material is dried under a vacuum for 2 days and then can be packaged in a 20 ml syringe.

It will be understood that various modifications may be made to the embodiments disclosed herein. For example, as used herein, the term "circular" is also intended to encompass gaskets that are not limited to circles, such as for example, oval-like gaskets, gaskets having at least one linear outer edge, etc. Additionally, the disclosed gaskets may include any regular or irregularly shape. Therefore, the above description should not be construed as limiting, but merely as exemplifications of disclosed embodiments. Those skilled in the art will envision other modifications within the scope and spirit of the claims appended hereto.

## Claims

1. A gasket for use with a surgical stapling apparatus, the gasket comprising a diisocyanate end-capped poly(ether ester).

2. The gasket according to claim 1, wherein the diisocyanate is selected from the group consisting of aromatic, aliphatic and acyclic isocyanates.

3. The gasket according to claim 1, wherein the diisocyanate is selected from the group consisting of 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), tetramethylxylylene diisocyanate, tetramethylene diisocyanate, hexamethylene diisocyanate, dimethyl diisocyanate, lysine diisocyanate, 2-methylpentane-1,5-diisocyanate, 3-methylpentane-1,5-diisocyanate or 2,2,4-trimethylhexamethylene diisocyanate, isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate and combinations thereof.

4. The gasket according to any preceding claim, wherein the poly(ether ester) further comprises at least one polyol and at least one polyacid, preferably wherein the at least one polyol is selected from the group consisting of polyalkylene oxides, polyvinyl alcohols, polysaccharides, and combinations thereof.

5. The gasket according to claim 4, wherein the at least one polyacid is an aliphatic polyacid, preferably wherein the aliphatic polyacid is selected from the group consisting of sebacic acid, azelaic acid, suberic acid, pimelic acid, adipic acid, glutaric acid, succinic acid, malonic acid, oxalic acid and combinations thereof.

6. The gasket according to any preceding claim, wherein the gasket is bioabsorbable, preferably wherein the gasket is absorbed in the body of from about 1 day to about 21 days.

7. The gasket according to any preceding claim, wherein the gasket is tear-resistant, and/or the gasket according to any preceding claim, wherein the gasket is elastic; or.the gasket according to any preceding claim, wherein the gasket is compliant; and/or the gasket according to any preceding claim, wherein the gasket is swellable; and/or the gasket according to any preceding claim, wherein the gasket is a curable material.

8. The gasket according to any preceding claim, wherein the gasket is UV curable; and/or. the gasket according to claim 1, wherein at least a portion of the gasket is porous.

9. The gasket according to any preceding claim, wherein the gasket further comprises a wound treatment material.

10. A gasket for use with a surgical stapling apparatus, the gasket comprising a pre-cured adhesive or sealant.

11. The gasket according to claim 10, wherein the gasket comprises a material selected from the group consisting of diisocyanate end-capped poly(ether esters), polyesters, polyamides, polyurethanes, polypeptides, proteins, nucleic acids, polysaccharides and combinations thereof.

12. A gasket for use with a surgical stapling apparatus, the gasket comprising:
a body portion having an upper surface and a lower surface, the body portion defining a central longitudinal axis extending between the upper surface and the lower surface, body portion comprising a diisocyanate end-capped poly(ether ester); and
a plurality of pores extending through the body portion, wherein each of the pores extends from the upper surface to the lower surface such that each pore defines an axis that is substantially parallel to the central longitudinal axis;
the body portion having an outer region disposed outwardly of the plurality of pores, wherein the outer region is substantially non-porous.

13. The gasket of Claim 12, wherein the diisocyanate is selected from the group consisting of: lysine diisocyanate, hexamethylene diisocyanate, and phenylene diisocyanate.

14. The gasket of Claim 12 or 13, wherein the body portion is dimensioned so that the plurality of pores is positioned at a staple line of the surgical stapling apparatus and the outer region is positioned outwardly of the staples fired by the surgical stapling apparatus.

15. The gasket of Claim 12 or 13, wherein the body portion has an outer terminal edge, the body portion being dimensioned to that the outer terminal edge is positioned outwardly of an outer perimeter of an anvil assembly of the surgical stapling apparatus.

16. The gasket of any of Claims 12 to 15, wherein the plurality of pores is formed by cutting the body portion; or the gasket of any of Claims 12 to 15, wherein the plurality of pores is formed by stamping.

17. The gasket of Claim 12 or 13, wherein the body portion includes an outer terminal edge, and an inner terminal edge defining an aperture therein, the aperture being dimensioned and arranged to receive a shaft of a surgical stapling apparatus.

18. The gasket of any of claims 12 to 17, wherein the body portion is substantially circular in shape; and/or the gasket of any of claims 12 to 17, wherein the body portion includes polyethylene glycol; and/or the gasket of any of claims 12 to 17, wherein the body portion is elastic; and/or.the gasket of any of claims 12 to 17, wherein the body portion is swellable.
